# EUROPEAN PATENT APPLICATION

(11) **EP 3 705 114 A1**
(43) Date of publication of application: **09.09.2020**
(21) Application number: 19382170.9
(22) Date of filing: 06.03.2019
(51) Int. Cl.: A61K 9/00

(54) **OPHTHALMIC COMPOSITION FOR TREATING DRY EYE AND SYMPTOMS THEREOF**

(71) Applicant: Avizorex Pharma, S.L., 08028 Barcelona (ES)
(72) Inventor: Galán Valdivia, Francisco Javier, 08028 Barcelona (ES)
(74) Representative: ZBM Patents ApS

(57) **Abstract**

It is provided an ophthalmic composition, particularly formulated as eye drops, comprising a non-ionic surfactant as active ingredient, wherein the non-ionic surfactant is present in an effective amount from 0.01 to 8 weight/volume % based on the total composition, for use in treating or preventing dry eye and/or for relieving one or more symptoms and/or signs of dry eye. The ophthalmic composition exerts a rapid effect in treating dry eye symptoms and signs while having very low incidence of side effects.

## Description

### FIELD OF THE INVENTION

The present invention refers to the field of medicine and particularly, to novel ophthalmic compositions in form of e.g. eye drops, for use in treating or preventing dry eye and/or for relieving one or more symptoms or signs of dry eye.

### BACKGROUND ART

Tears of the eye or tear film has a three layered structure made up of an oil layer, a water layer and a mucin layer. Dry eye syndrome or dry eye disease (hereinafter referred to as "dry eye") come in a wide range of concepts, and in many cases, its cause is not known. Thus, dry eye is defined, rather than a disease, as an abnormal condition of eyes resulting from an unstable tear film caused by a decrease or change in quantity or quality of tears and by tear film breaking up time faster than normal.

Dry eye is a common eye disease. It affects 5-34% of people to some degree depending on the population looked at. Among older people it affects up to 70%.

Dry eye occurs when either the eye does not produce enough tears or when the tears evaporate too quickly. This can result from contact lenses use, meibomian gland dysfunction, allergies, pregnancy, Sjögren's syndrome, vitamin A deficiency, LASIK surgery, and certain medications such as antihistamines, some blood pressure medication, hormone replacement therapy, and antidepressants. Chronic conjunctivitis such as from tobacco smoke exposure or infection may also lead to the condition. Diagnosis is mostly based on the symptoms, though a number of tests may be used.

Treatment of dry eye focuses on allowing for the maintenance of at least a predetermined volume of tears by a conservative method such as replenishing artificial tear eye drops or blocking the nasolacrimal ducts temporarily or eternally. However, with the expansion of treatment concept, the recent trend of dry eye treatment moves toward active treatment using drugs which promote the secretion of lacrimal fluid even for mild dry eye, rather than passive treatment for the purpose of disease alleviation.

In keeping with this trend, the use of immunosuppressive agents to treat inflammation associated to dry eye becomes more frequent. Immunosuppressive agents include cyclosporine, sirolimus, tacrolimus and their derivatives. Among them, one of the commercially available drugs is cyclosporine A 0.05 % emulsion form eye drops (RESTASIS from Allergan). This is known as being effective in treating dry eye and its related keratoconjunctival epithelial disorder, but at the same time, is known as causing many side effects. The most common abnormal reaction is burning sensation in eyes, and it has also been reported conjunctival hyperaemia, eye discharge, epiphora, eye pain, foreign body sensation, pruritus, stabbing sensation, vision impairment (often blurred vision). According to the assay whose author is Lee JE et al., 2007, as a result of contact with 0.05 % cyclosporine for 10 minutes, increased apoptosis and reduced cell viability was seen, and to use without toxic effect of corneal epithelial cells, the duration of exposure to 0.05 % cyclosporine should be less than 10 minutes (Lee JE et al., 2007), and cell viability significantly reduced at 0.05 % or at a greater amount of cyclosporine (Garweg JG et al., 2006).

In addition to cyclosporine, the use of an excessive amount of immunosuppressive agents causes side effects such as anemia, leukopenia, thrombocytopenia, and hair loss. However, in keeping with the recent trend, for treatment of severe dry eye as well as clinical symptoms of discomfort even in mild cases, immunosuppressive agents are actively used. Further, elderly patients, who are the most frequent patients with dry eye, use many drug types and frequent doses, and it is known that these factors reduce patient compliance and treatment effect.

Other dry eye approved products are Lifitegrast 5 % ophthalmic solution (XIIDRA from Shire) and Diquafosol 3 % ophthalmic solution (DIQUAS from Santen) in the Asian market, but they present limited efficacy.

In spite of the efforts in the field, the problem of dry eye remains very extended and unsolved being an unmet medical need. Thus, it would be desirable to provide new improved treatments for dry eye.

### SUMMARY OF THE INVENTION

One problem to be solved by the present invention may be seen as related to the provision of ophthalmic compositions with improved properties for the treatment of dry eye and symptoms thereof.

The solution is based on the provision of an ophthalmic composition with a rapid effect in treating dry eye symptoms and signs, and with a rapid onset of action and very low incidence side effects.

Accordingly, a first aspect of the invention relates to an ophthalmic composition comprising a non-ionic surfactant as active ingredient, wherein the non-ionic surfactant is present in an effective amount from 0.01 to 8 weight/volume % based on the total composition, for use in treating or preventing dry eye and/or for relieving one or more symptoms and/or signs of dry eye.

The terms used herein have the same meaning as commonly understood by a person skilled in the art. Nevertheless, the terms "non-ionic surfactant" and "dry eye" are explained below in the detailed description of the invention.

Unless otherwise indicated, percentages provided for the ingredients of the ophthalmic composition of the present invention are weight/volume percentages (w/v %).

The composition of the invention is directed to the treatment of dry eye but it is also directed to the relief or alleviation of symptoms or signs of dry eye. Such symptoms can be present in a pre-clinical dry eye state or also without developing dry eye at the end i.e. in transitory discomforts not ending in dry eye disease, such as in circumstantial conditions with ocular surface dryness or ocular discomfort.

The working examples herein provided demonstrate that the ophthalmic composition of the invention exerts a rapid effective effect on dry eye symptoms and signs, as shown in a phase I/II double-masked clinical study in 50 human subjects with mild-to-severe dry eye disease. Remarkably, the working examples show that the composition of the invention reduces more than 20 points in SANDE score, which would be considered a clinically significant improvement.

Non-ionic surfactants are widely used in the art as solubilizers of poorly water soluble substances as well as emulsifiers to form stable emulsions, microemulsions and nanoemulsions. For that reason non-ionic surfactants are included in pharmaceutical compositions as "non-active ingredient" since no therapeutic activities are expected due to its use.

Surprisingly, the inventors have obtained unexpected results from an eye drops formulation containing certain amount of polyoxyl 35 castor oil, a non-ionic surfactant acceptable for its use in ophthalmic products. When administered in patients suffering ocular discomfort related to ocular surface dryness, it provides a rapid relief for dry eye symptoms measured by a reduction in SANDE scores. It also improves the ocular surface integrity and health measured by a decrease in corneal and conjunctival staining.

Ocular surface integrity and health depends on a stable tear film which is influenced by volume and quality of tears as well as appropriate blinking rate. When the ocular surface suffers from excessive tears evaporation, decreased tear production or other undesired conditions, the ocular surface integrity and mucin layer are affected, appearing dried small areas which produce discomfort and certain level of inflammation.

Without being bound to the theory, it is believed that a non-ionic surfactant in the appropriate amount will produce a decrease in the superficial tension of tears and additionally, a decrease in the contact angle between the tear film and the ocular surface epithelium that is regularly modified during blinking. This will lead to a significant improvement in the tear film ability to spread over the ocular surface and an improvement in wetness (or wettability) and health, alleviating undesired dry eye symptoms. Overall, the ocular homeostasis is significantly improved. Thus, the non-ionic surfactant of the composition of the invention is functioning as active ingredient improving the ocular surface wetness by helping the distribution of fluid homogeneously over the eye surface made by the rhythmic lid closure. This is opposite to the prior art, wherein non-ionic surfactants in ophthalmic compositions are used as non-active ingredients (i.e. as emulsifier or solubilizer agent).

The invention also provides an ophthalmic composition consisting of a non-ionic surfactant as sole active ingredient, a buffer, a tonicity agent and a pH adjusting agent, wherein the non-ionic surfactant is present in an effective amount from 0.01 to 8 w/v % based on the total composition.

### DESCRIPTION OF THE DRAWINGS

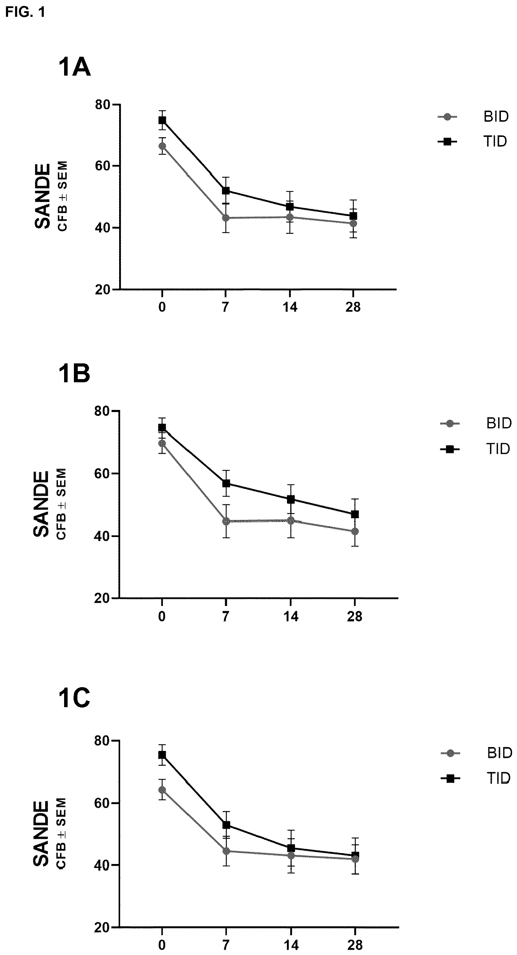

FIG. 1. Effect of Composition-A BID and TID dose regimen on SANDE score. Horizontal axis = day of evaluation. CFB ± SEM = SANDE mean scores ± standard error of mean. BID = twice a day. TID = three times a day. See working Example 4.1 herein for further details. FIG. 1A shows the treatment effect of Composition-A in SANDE total score, P value < 0.0001. FIG. 1B shows the treatment effect of Composition-A in SANDE frequency score, P value < 0.0001. FIG. 1C shows the treatment effect of Composition-A in SANDE severity score, P value < 0.0001.

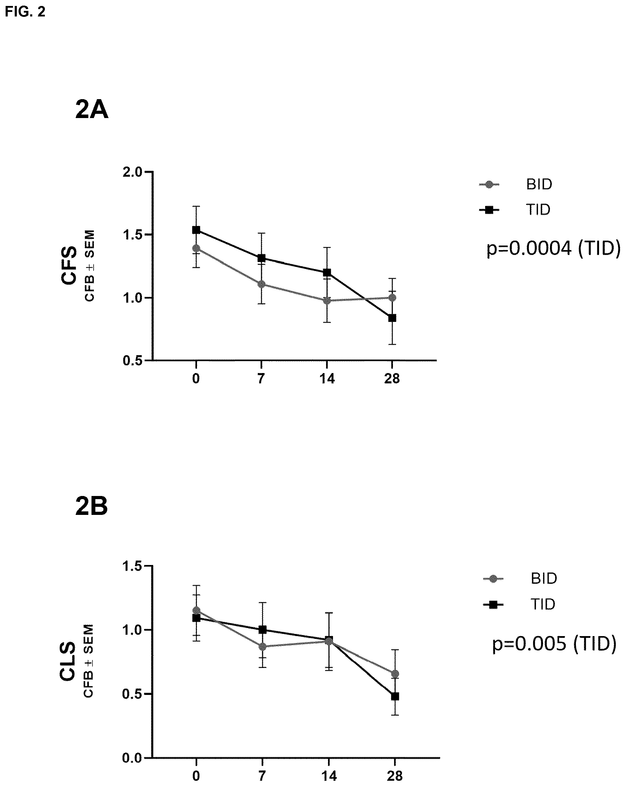

FIG. 2. Effect of Composition-A BID and TID dose regimen on corneal and conjunctival staining. Horizontal axis = day of evaluation. CFB ± SEM = staining mean scores ± standard error of mean. BID = twice a day. TID = three times a day. See working example 4.2 herein for further details. FIG. 2A shows effect of Composition-A in Corneal Fluorescein Staining (CFS), P value = 0.0004 for TID. FIG. 2B shows effect of Composition-A in Conjunctival Lissamine Staining (CLS), P value = 0.005 for TID.

### DETAILED DESCRIPTION OF THE INVENTION

### Ophthalmic composition for use in treating or preventing dry eye and/or for relieving one or more symptoms and/or signs of dry eye

In one aspect, an ophthalmic composition is provided comprising a non-ionic surfactant as active ingredient, wherein the non-ionic surfactant is present in an pharmaceutical effective amount, for use in treating or preventing dry eye and/or for relieving one or more symptoms and/or signs of dry eye. A "pharmaceutically effective amount" is that dose required to prevent, inhibit the occurrence, or treat a disease state (i.e. alleviate a symptom to some extent, preferably all of the symptoms). The pharmaceutically effective amount generally depends on the type of disease, the composition used, the route of administration, the type of mammal being treated, the physical characteristics of the specific mammal under consideration, concurrent medication, and other factors that those skilled in the medical arts will recognize.

An aspect of the invention relates to an ophthalmic composition comprising a non-ionic surfactant as active ingredient, wherein the non-ionic surfactant is present in an effective amount from 0.01 to 8 w/v % based on the total composition, for use in treating or preventing dry eye and/or for relieving one or more symptoms and/or signs of dry eye. This aspect can be alternatively formulated as the use of the ophthalmic composition as defined above for the manufacture of a pharmaceutical product, a medicament or a veterinary product, for the prevention and/or treatment of dry eye and/or for relieving one or more symptoms and/or signs thereof. This may be also alternatively formulated as a method for the prevention and/or treatment of dry eye and/or for relieving one or more symptoms and/or signs of dry eye in a mammal, including a human, comprising administering to said mammal in need thereof an effective amount of the ophthalmic composition as defined in this aspect of the invention.

### Dry eye

The term "dry eye" in this description, is understood according to the recently reviewed definition of dry eye provided by TFOS DEWS II. Dry eye disease (DED), also called "dry eye syndrome" or "keratoconjunctivitis sicca", is a multifactorial disease of the ocular surface characterized by a loss of homeostasis of the tear film, and accompanied by ocular symptoms, in which tear film instability and hyperosmolarity, ocular surface inflammation and damage, and neurosensory abnormalities play etiological roles (Craig JP et al., 2017). Dry eye occurs when either the eye does not produce enough tears or when the tears evaporate too quickly.

### Causes of dry eye

Any abnormality of any one of the three layers of tears produces an unstable tear film, resulting in symptoms of dry eye. As said before the primary cause of dry eye is a decreased tear production or excessive evaporation. Causes include idiopathic, congenital alacrima, lacrimal gland ablation, lacrimal gland dysfunction or damage, and sensory denervation.

In a particular embodiment, dry eye can be caused or associated to one or more diseases or conditions selected from the group consisting of:
- Keratoconjunctivitis sicca, which is chronic, bilateral desiccation of the conjunctiva and cornea due to an inadequate tear film;
- Keratoconjunctival epithelial disorder, which is caused by tear deficiency in the mucus layer, and serious corneal damage. Particularly, categories of keratoconjunctival epithelial disorder include dry eye, corneal epithelial defect, conjunctival epithelial defect, corneal epithelial erosion, reduced corneal thickness, corneal infiltrate, corneal perforation or corneal epithelial exfoliation. Keratoconjunctival epithelial disorder results in corneal ulcer, keratitis, conjunctivitis, superficial punctate keratopathy, keratoconjunctivitis sicca, superior limbic keratoconjunctivitis, filamentary keratitis, corneal ulcer and infectious eye diseases of corneal and conjunctival epithelium. The keratoconjunctival epithelial disorder may be caused by injury in eye, microsurgery or hard contact lens wear;
- Reduced lacrimal fluid secretion;
- Stevens-Johnson syndrome;
- Tear deficiency;
- Ocular hyperemia;
- Tear film instability, such as reduced lipid layer caused by e.g. meibomian gland dysfunction (MGD);
- Eye edema;
- Infiltration of the lacrimal glands by sarcoidosis or tumors;
- Post-radiation fibrosis of the lacrimal glands;
- Sjogren's syndrome, an immune system disorder characterized by inflammation and dryness of the mouth, eyes and other mucous membranes. This disorder damages the lacrimal glands and affects tear production;
- Collagen vascular diseases, including relapsing polychondritis, rheumatoid arthritis, granulomatosis with polyangiitis, and systemic lupus erythematosus;
- Diabetes; and
- Abnormalities of the lipid tear layer caused by blepharitis and rosacea, and abnormalities of the mucin tear layer caused by vitamin A deficiency, trachoma, diphtheric keratoconjunctivitis, mucocutaneous disorders and certain topical or systemic medications.

More particularly, dry eye or the symptoms and/or signs thereof, are caused or associated to a disease or condition selected from the group consisting of: keratoconjunctivitis sicca, keratoconjunctival epithelial disorder, reduced lacrimal fluid secretion, Stevens-Johnson syndrome, Sjogren's syndrome, tear deficiency, ocular hyperemia and, tear film instability.

In a particular embodiment, dry eye is caused or associated to one or more factors selected from the group consisting of:
- Aging: Tear production decreases with age. About 75 % of individuals over the age of 65 suffer from dry eye symptoms;
- Digital device use (TV or computers): screen time has increased exponentially and is probably the main reason the latest studies on dry eye show a significant increase in the condition in younger populations. It is called visual display terminals (VDT) operation-related dry eye;
- Environmental factors: people tend to have dry eye symptoms when they are exposed to smoke, air pollution, high altitude, sunny, windy, cold or dry air conditions;
- Contact lens wear: contact lens wear can dramatically increase tear evaporation and damage the corneal surface epithelium, causing dry eye discomfort;
- Hormonal changes in women: various hormonal changes associated with pregnancy, oral contraceptives and menopause can contribute to dry eye symptoms;
- Medications: the list of medications having dry eyes as a side effect is very extensive; among them: isotretinoin, sedatives, diuretics, tricyclic antidepressants, antihypertensives, oral contraceptives, antihistamines, nasal decongestants, beta-blockers, phenothiazines, atropine, chronic administration of other neurotropic drugs, pain relieving opiates such as morphine, anticholinergic medications that also cause dry mouth besides dry eye. These drugs can cause or worsen this condition;
- Refractive or other ocular surgery, e.g. laser vision corrective surgery, cataract, glaucoma or retinal surgery: dry eyes also occurs or gets worse after LASIK and other refractive surgeries, in which the corneal nerves are cut during the creation of a corneal flap. The corneal nerves stimulate tear secretion. Dry eye caused by these procedures usually resolves after several months, but it can be permanent;
- Thermal or chemical burns;
- An eye injury or other problem with the eyes or eyelids, such as bulging eyes or a drooping eyelid: Disorders of the eyelid can impair the complex blinking motion required to spread tears; and
- Allergic, bacterial, amoebal and viral keratoconjunctivitis.

More particularly, dry eye is caused or is associated to a factor selected from the group consisting of contact lenses use, digital device use, refractive or other ocular surgery, chronic administration of neurotropic drugs, allergic, amoebal and viral keratoconjunctivitis, aging, hormonal changes, and environmental factors.

### Dry eye symptoms and signs

The composition of the invention is also directed to relieve or alleviate dry eye symptoms and/or signs. Such symptoms can be present in a pre-clinical dry eye state or also without developing dry eye at the end e.g. as a transient condition. It is recognized that symptoms consistent with dry eye, but in the absence of clinical signs, especially when the symptoms are intermittent, might indicate a pre-clinical dry eye state, or a scenario of emerging episodic dry eye.

Thus, the composition of the invention is useful in the treatment or prevention of transitory discomforts, not ending in dry eye disease; i.e. in circumstantial conditions accompanied by ocular surface dryness or ocular discomfort.

It can also occur that patients exhibit signs of ocular surface disease, but reporting no symptoms of discomfort. Corneal nerve damage secondary to longstanding dry eye is a recognized phenomenon and the reduced corneal sensitivity can mask discomfort. The dysfunctional sensation is a function of the underlying disease process. Other forms of corneal disease exist, where corneal sensation is reduced, and these should also be managed accordingly.

Ocular surface changes in the absence of presenting symptoms may be noted during a preoperative examination for cataract or refractive surgery, e.g., and represent an early disease state that might place the patient at risk of developing symptomatic dry eye following the surgical event. In this case the composition of the invention is administered to the patient to prevent the onset of dry eye or symptoms and signs thereof.

Craig JP et al., 2017 describes symptoms and signs of dry eye, and it is incorporated herein by reference.

Dry eye symptoms can range from mild and occasional to severe and continuous. Most people who have dry eyes experience mild irritation with no long-term effects. However, if the condition is left untreated or becomes severe, it can produce complications that can cause eye damage, resulting in impaired vision or (rarely) in the loss of vision. In mild cases, a patient may experience burning, hot sandy, gritty itching feelings of dryness, and persistent irritation. In severe cases, vision may be substantially impaired. The resultant damage to the eye increases discomfort and sensitivity to bright light. Both eyes are usually affected. Because blinking coats the eye with tears, symptoms are worsened by activities in which the rate of blinking is reduced due to prolonged visual attention. These activities include prolonged reading, computer usage, driving, or watching television. Symptoms increase in windy, dusty or smoky (including cigarette smoke) areas, in dry environments high altitudes including airplanes, on days with low humidity, and in areas where an air conditioner (especially in a car), fan, heater, or even a hair dryer is being used. Symptoms reduce during cool, rainy, or foggy weather and in humid places, such as in the shower. Symptoms due to a refractive surgery, may be transient, e.g. from six weeks to six months or more following surgery.

In particular embodiments, the composition of the invention is used for relieving or alleviating one or more symptoms, particularly:
- Dryness;
- Burning;
- Irritation;
- Itching;
- Scratchy;
- Stinging;
- Tired eyes or easily fatigued eyes;
- Pain or soreness in or around eyes;
- Blurry/fluctuating vision;
- Eyes redness;
- Foreign body sensation feeling that something such as a speck of dirt is in the eye, gritty, sandy feeling;
- Impaired blinking motion;
- A stringy discharge from the eyes. Although it may seem strange, dry eye can cause the eyes to water. This can happen because the eyes are irritated. One may experience excessive tearing (epiphora) in the same way as one would if something got into the eye. These reflex tears will not necessarily make the eyes feel better. This is because they are the watery type that are produced in response to injury, irritation, or emotion. They do not have the lubricating qualities necessary to prevent dry eye, interfering with eye comfort;
- Discomfort;
- Sensitivity to light;
- Excessive tearing;
- Contact lens discomfort or intolerance;
- Scarring of the cornea; and
- Inflammation of the eye surface.

Although it appears that dry eye may result from a number of unrelated pathogenic causes, all presentations of the complication share a common effect, that is the breakdown of the pre-ocular tear film, which results in exposure of the ocular surface, dehydration, and cytokine production resulting in many symptoms outlined above. Thus, in particular embodiments, the composition of the invention is used for relieving or alleviating one or more signs of dry eye, particularly:
- Reduced tear volume and tear poor quality (these aspects are usually tested with the Schirmer Test);
- Reduced tear break-up time (TBUT): there are three layers of tears: the mucin layer, the aqueous layer and the outermost lipid layer. The lipid layer is responsible for the stability of the tear film over the cornea. TBUT is caused by deficiency of the lipid layer which reduces the stability of the tear film thus "breaking" it up. The causes of reduced lipid layer are many, the most common being meibomian gland dysfunction (MGD);
- Reduced tear meniscus;
- Presence of inflammatory cytokines in the tear film;
- Presence of small dried areas in the cornea and conjunctiva (tested by e.g. Corneal and Conjunctival Staining): small parts on the eye are dried when the eye surface is not well lubricated due to lack of mucus layer, deficiency in tears volume or reduced blinking rate. When a dye is instilled to the eye those dried areas appear as stained spots.

In a particular embodiment, dry eye symptoms and/or signs are selected from the group consisting of: foreign body sensation, burning, stinging, itching, pain, dryness, redness of the eye, reduced tear volume, tear poor quality, reduced tear break-up time, presence of small dried areas in the cornea and conjunctiva detected by staining.

The therapeutic benefit of the composition of the invention is the amelioration of one or more of the symptoms and/or signs described above.

### Non-ionic surfactants

Non-ionic surfactant refers to a kind of molecule not undergoing ionization when being dissolved in water. According to their different hydrophilic groups, it can be divided into polyoxyethylene and polyols categories.

A non-ionic surfactant has a high stability and is not easily affected by the acid-base or strong electrolytes. Moreover, it also has good compatibility with anionic, cationic surfactants or amphoteric surfactants and can be used in formulation. The non-ionic surfactant are not in the ionic state in the solution, thereby having high stability and being less susceptible to the effect of strong electrolyte inorganic salts as well as acid and alkalis; it has excellent compatibility with other types of surfactants and has excellent solubility (which vary depending on different structures) in both water and organic solvents.

In a particular embodiment, the non-ionic surfactant is selected from the group consisting of an oleoyl macrogolglyceride, a linoleoyl macrogolglyceride, a caprylocaproyl polyoxylglyceride, polyoxyl 35 castor oil, polyoxyl 35 hydrogenated castor oil, polyoxyl 40 castor oil, polyoxyl 40 hydrogenated castor oil, and a condensation product of ethylene oxide with 12-hydroxystearic acid.

More particularly, the non-ionic surfactant is polyoxyl 35 castor oil. Polyoxyl 35 castor oil (CAS-No: 61791-12-6), also known as Cremphor® EL, or Kolliphor® EL, is a non-ionic solubilizer and emulsifier made by reacting castor oil with ethylene oxide in a molar ratio of 1:35. The main component is glycerol polyethylene glycol ricinoleate. Together with fatty acid esters of polyethylene glycol, this forms the hydrophobic part of the product. The smaller hydrophilic part consists of free polyethylene glycols and ethoxylated glycerol. It is commonly used for the production of semi-solid and liquid formulations of water-insoluble actives and other hydrophobic compounds.

The amount of non-ionic surfactant in the ophthalmic composition will depend upon factors such as the efficacy of non-ionic surfactant at different concentrations, the compatibility of the non-ionic surfactant with other ingredients in the composition, the ability of biological target to accept various amounts of non-ionic surfactant, combinations thereof or the like. Particularly, the non-ionic surfactant is present in an amount from 0.01 to 8 w/v %. In a particular embodiment, the non-ionic surfactant is present in an amount from 0.5 to 5 w/v %. More particularly, it is present at 0.5, 1, 1.5, 2, 2.5, 3, 3.5, 4, 4.5 or 5 w/v %. Particularly, the non-ionic surfactant is present at 3 w/v %.

### Formulations, route of administration and dosage

In a particular embodiment, the ophthalmic composition is formulated as aqueous solution, which is easily administered by the patient by means of instilling one or two drops of the solution in the affected eyes. However, the compositions may also be suspensions, viscous or semi-viscous gels, dissolved ophthalmic solutions or other types of solid or semi-solid compositions e.g. an ophthalmic cream.

In a particular embodiment the ophthalmic composition further comprises a buffer. The buffer may be selected from the group consisting of a phosphate buffering agent, a borate buffering agent, a citrate buffering agent, a tartrate buffering agent, an acetate buffering agent (e.g., sodium acetate) but is not limited thereto. Particularly, the buffer is a phosphate buffering agent (NaH₂PO₄-2H₂O).

Thus, in the case of being prepared as an aqueous solution, the ophthalmic solution may include other various additives known in the art, e.g., a buffering agent, a tonicity adjusting agent, a stabilizing agent, a pH adjusting agent, a thickening agent, a preservative, a chelating agent, a solubilizing agent and a solvent, so long as the object of the present disclosure is not hindered.

The ophthalmic pharmaceutical compositions of the present invention will generally be formulated as sterile aqueous solutions. These compositions are also formulated so as to be compatible with the eye to be treated with the compositions. The ophthalmic compositions intended for direct application to the eye will typically be formulated so as to have a pH and tonicity that are compatible with the eye. It is also contemplated that the compositions can be suspensions or other types of solutions. The ophthalmic compositions will typically have a pH in the range of 4 to 9, particularly 5.5 to 8.5, and most particularly 5.5 to 8.0. Particularly desired pH ranges are 6.0 to 7.8, more particularly 6.4 to 7.2 or 7.5, and even more particularly 6.8 to 7.2. The pH adjusting agent may be selected from the group consisting of hydrochloric acid, acetic acid, phosphoric acid, sulfuric acid, sodium hydroxide, potassium hydroxide, monoethanolamine, ammonia water and ammonium hydroxide, but is not limited thereto.

The tonicity adjusting agent may be selected from the group consisting of sugars such as sorbitol, glucose, erythritol and mannitol, polyhydric alcohols such as glycerin, polyethylene glycol and polypropylene glycol, and salts such as sodium chloride, but is not limited thereto. Particularly, the tonicity adjusting agent is sodium chloride.

The thickening agent may be selected from the group consisting of hydroxyethylcellulose, hydroxypropylcellulose, methylcellulose, hydroxypropylmethylcellulose (hypromellose) and carboxymethylcellulose, polyvinylalcohol, carbomer, povidone, poloxamer, hyaluronic acid, polycarbophil and its salt, but is not limited thereto. Particularly, the thickening agent is hypromellose.

The chelating agent may be selected from the group consisting of sodium edetate, sodium citrate and condensed sodium phosphate, but is not limited thereto.

The solubilizing agent or solvent may be selected from purified water, glycerin, DMSO, DMA, N-methylpyrrolidone, ethanol, benzylalcohol, isopropylalcohol, various molecular weights of polyethyleneglycol or propylene glycol, but is not limited thereto. There may be some overlaps between substances that can be used for the solvent or the solubilizing agent, and any substance may be used for any one of the solvent and the solubilizing agent, then if the substance acts as a solvent in the preparation, it is regarded as a solvent, and if the substance does not act as a solvent, it is regarded as a solubilizing agent.

Topical ophthalmic products may be packaged in multidose form. Preservatives may thus be required to prevent microbial contamination during use. Suitable preservatives include: chlorobutanol, methyl paraben, propyl paraben, phenylethyl alcohol, edetate disodium, sorbic acid, polyquaternium, benzalkonium chloride, benzethonium chloride, alkyl paraoxybenzoate such as methyl paraoxybenzoate and ethyl paraoxybenzoate, benzyl alcohol, sorbic acid and its salt, thimerosal, benzododecinium bromide, oxychloro complex and chlorobutanol, but is not limited thereto. Such preservatives are typically employed at a level of from 0.001 to 5.0% w/v.

Unit dose compositions of the present invention will be sterile, but typically unpreserved. Such compositions, therefore, generally will not contain preservatives. Thus, the ophthalmic compositions of the present invention may also be provided preservative free and packaged in unit dose form or multidose form suitable for composition preservative free. In a particular embodiment, the composition is formulated as multidose ophthalmic composition preservative free. In another embodiment, the composition is formulated in single dose vials.

According to a particular embodiment, the ophthalmic composition of the present invention is formulated as eye drops and includes 0.01 to 8 w/v % of a non ionic surfactant as active ingredient, particularly polyoxyl 35 castor oil, 0.01-2 w/v % of a thickening agent, the remainder of a buffering agent and a tonicity adjusting agent, on the basis of the total weight of the composition.

In one aspect, the ophthalmic composition consists of a non-ionic surfactant as sole active ingredient, a buffer, a tonicity adjusting agent and a pH adjusting agent, wherein the non-ionic surfactant is present in an effective amount from 0.01 to 8 w/v % based on the total composition. In a particular embodiment, the non-ionic surfactant is polyoxyl 35 castor oil. In a particular embodiment the composition further comprises a preservative, and/or a thickening agent and/or a chelating agent.

In a particular embodiment, the ophthalmic composition is formulated as eye drops for direct instillation to the eye. In one embodiment these eyedrops have a volume of between 25 and 50 microliters.

As discussed, particularly, the composition is administered topically to the eye, but other administration routes other than directly to the eye can also be used. The precise dosage and administration schedule to be employed in the formulation will also depend on the route of administration.

The composition of the invention is formulated in a particular embodiment as artificial tears, that means that it can be sold over-the-counter for symptomatic relief without doctor's prescription.

In treatment and/or prevention of mammals, in particular, humans, dosage of the ophthalmic composition according to the present disclosure may be generally determined by those working in the medical industry or those having related ordinary skill. For example, when the composition according to the present disclosure is used as eye drops to an adult patient with dry eye, a particular dosage of the ophthalmic composition may be administration at a dose of 1 to 4 drops (about 0.025 to 0.1 mL) of eye drops from 1 to 10 times per day, but is not limited thereto, and those working in the medical industry or those having related ordinary skill may determine a most optimal real dosage based on not only the age, weight, gender and reaction of a patient to treat, but also the condition that is expected with this treatment.

In a particular embodiment, the composition is administered one time a day. In another embodiment, the composition is administered more than one time a day, particularly from 1 to 10 times a day and more particularly, 2 or 3 times a day.

In a particular embodiment, the ophthalmic composition is administered at a dose at least 1 drop to each eye from 1 to 6 times per day, Particularly, a dose of 1 drop to each eye is administered from 1 to 6 times a day.

As discussed before, the non-ionic surfactant is present in the ophthalmic composition in an amount from 0.01 to 8 w/v %. In a particular embodiment, the non-ionic surfactant is present in an amount from 0.5 to 5 w/v %. More particularly, it is present at 0.5, 1, 1.5, 2, 2.5, 3, 3.5, 4, 4.5 or 5 w/v %. Particularly, the non-ionic surfactant is present at 3 w/v %. Assuming an amount of non-ionic surfactant of 1 % (equivalent to 10 mg/ml), that each drop contains 50 microliters, and that the composition is administered 2-3 times per day in both eyes, the equivalence in dose/day is the following:
BID (twice a day): 200 microliters per day (= 0.2 ml). 10 mg/ml x 0.2 ml = 2 mg per eye and per day.
TID (three times a day): 300 microliters per day (= 0.3 ml). 10 mg/ml x 0.3 ml = 3 mg per eye and per day.

The composition may be administered for a prolonged period of time as needed. Given that dry eye and/or symptoms thereof are often chronic conditions, the dosages may be administered on a daily basis during a long period resulting in a chronic administration. Accordingly, administration may be continued for more than 4 weeks on a daily basis, or alternatively, administration may be continued for more than 4 weeks but not on a daily basis. The precise schedule can be determined in accordance with the severity of the chronic condition. In a particular embodiment the composition is administered for 4 to 24 weeks.

The composition of the present invention is prepared by methods known by the skilled in the art. Briefly, solutions of each component are prepared at suitable temperature and stirring, and then the solutions are mixed to obtain an homogeneous solution. An example of manufacturing process is the following: A thickening agent (e.g. hypromellose) is dissolved at a suitable temperature such as 90 °C, in a suitable reactor. Temperature and stirring are kept until the thickening agent is completely dissolved. A buffer stock solution is prepared using a beaker and a magnetic stirrer. Stirring is kept e.g. at 1200 rpm, until complete dissolution. A non-ionic surfactant is heated at a suitable temperature (e.g. 60 °C) and the solution is stirred until complete dissolution. The non-ionic surfactant solution is added stepwise and under continuous stirring into the buffer solution. Then, pH is adjusted to around 7.0. Finally, the solution is adjusted to the desired volume with purified water. The latter solution is added under continuous stirring into the thickening agent solution and a suitable amount of purified water previously cooled is added. The mixture is stirred during e.g. 20 minutes, and the pH is adjusted again to around 7.0. If a second active ingredient is added at the composition, it can be prepared together with the non-ionic surfactant or with the buffer solution depending on the thermal stability of the second active ingredient. The final formulation is submitted to a sterile filtration process. The formulation is then transferred into a suitable vessels such as stainless steel pressure vessel, weighed and the final volume is adjusted with purified water. The resulting ophthalmic solutions are packaged into suitable vials e.g. single dose low-density polyethylene (LDPE) vials. With a syringe and a needle and in a vertical laminar flow booth the vials are filled using the formulation stored on the sterile containers. Every group of doses are packed into packets which are then thermally sealed.

The packaging for the composition of the present invention particularly is made from a material that is relatively impermeable with respect to the composition. The choice of material will in part depend on the desired product shelf life; i.e., the longer the desired shelf life, the more impermeable the material needs to be. The compositions of the present invention are particularly packaged in unit dose containers which are then sealed into laminated foil pouches. The manufacture and filling of such unit dose containers are known in the art (generally referred to as "form, fill and seal"). Multiple unit dose containers may be packaged in each laminated foil pouch.

When an amount, concentration, or other value or parameter is given as either a range, particular range, or a list of upper preferable values and lower preferable values, this is to be understood as specifically disclosing all ranges formed from any pair of any upper range limit or particular value and any lower range limit or particular value, regardless of whether ranges are separately disclosed. Where a range of numerical values is recited herein, unless otherwise stated, the range is intended to include the endpoints thereof, and all integers and fractions within the range. It is not intended that the scope of the invention be limited to the specific values recited when defining a range.

### Second active ingredient

In a particular embodiment the ophthalmic composition further comprises a second active ingredient. Particularly, the second active ingredient is selected from the group consisting of a Transient Receptor Potential cation channel subfamily M member 8 (TRPM8) agonist, and hyaluronic acid or a salt thereof.

In a particular embodiment, the second active ingredient is a TRPM8 agonist and is present in an amount from 0.0001 to 0.1 w/v %. More particularly, it is present in an amount from 0.0002 to 0.005 w/v %.

In a particular embodiment, the second active ingredient is hyaluronic acid of a salt thereof. Particularly, it is present in an amount from 0.01 to 1 w/v %.

The TRPM8 receptor or Transient Receptor Potential cation channel subfamily M member 8, also known as Cold and Menthol Receptor 1 or CMR1, is a protein that is coded by the TRPM8 gene in humans. The human TRPM8 gene is located in chromosome 2 in the 2p37.1 region. The TRPM8 receptor has six trans-membrane segments, with the C and N terminal ends on the cytoplasmic side. Four subunits tetramerize to form active channels.

TRPM8 is an ion channel that, after activated, allows sodium ions (Na+) and calcium ions (Ca2+) to enter the cell, thus generating depolarization of said cell, leading to a change in the membrane potential. The TRPM8 protein is expressed in sensory neurons and is activated by cold temperatures (approximately below 26 °C), by chemical agents, such as menthol, and by voltage.

In the present invention, "TRPM8 receptor agonist" is defined as any molecule binding specifically to the TRPM8 receptor and that, upon binding, can cause an increase in the activity of the TRPM8 channel, i.e., that increment sodium and calcium flow through the channel causing a cell depolarization. These agonists increase the stimulation of tear secretion by cold-sensitive fibers.

Examples of the TRPM8 receptor agonists adequate for use in this invention include, without limitation, the molecules: CPS369 (N-(p-menthane-3 carbonyl)-D-alanine ethyl ester), CPS368 (N-(p-menthane-3 carbonyl)-D-alanine methyl ester), CPS125 ((1R,2S,5R)-5-methyl-2-propan-2-yl-N-[4-(pyrimidin-2-ylsulfamoyl)phenyl]cyclohexane-1-carboxamide), Frescolat® MGA-2 Isomer (Menthone glycerol ketal), Frescolat® ML ((-)-Menthyl lactate), Coolant Agent 10 ((-)-Menthoxypropane-1,2-diol), Coolact P® ((-)-Isopulegol), Coolact 38D® ((+)-cis & (-)-trans p-Menthane-3,8-diol Ratio - 62:38), (-)-Cubebol, "Hasegawa's Cooling Compound" (N-substituted alkoxy p-menthane-3 carboxamide), "IFF's New GRAS Cooling Material" (N,N-Dimethyl menthyl succinamide), Icilin (AG-3-5, [1-[2-hydroxyphenyl]-4-[2-nitrophenyl-]-1,2,3,6-tetrahydropyrimidine-2-one]), 5-methyl-4-(1-pyrrolidinyl)-3-[2H]-furanone, "Faintly mint-like" (4,5-dimethyl-3-(1-pyrrolidinyl)-2-[5H]-furanone), 4-methyl-3-(1-pyrrolidinyl)-2-[5H]-furanone, WS-3 (N-ethyl-p-menthane-3-carboxamide), WS-5 (ethyl 3-(p-menthane-3-carboxamide), WS-11 (2-isopropyl-5-methyl-cyclohexanecarboxylic acid (2-hydroxy-1, 1-dimethyl-ethyl acid)-amide), WS-12 (2-isopropyl-5-methylcyclohexanecarboxylic acid (4-methoxyphenyl)-amide), WS-14 (2-isopropyl-5-methyl-cyclohexanecarboxylic acid tertbutylamide), WS-23 (2-isopropyl-N-2,3-trimethylbutyramide), WS-30 (2-isopropyl-5-methyl-cyclohexanecarboxylic acid ester 2,3-dihydroxy-propyl), WS-148 (1-(di-sec-butyl-phosphinoyl)-heptane), Geraniol, Linallol, Eucalyptol, Hydroxyl-citronellal, PMD-38 (p-menthane-3,8-diol), specific TRPM8 agonist antibodies and siRNAs, and constitutively active variants of TRPM8, or combinations thereof.

In a particular embodiment, the second active ingredient is the TRPM8 agonist 2-isopropyl-5-methyl-cyclohexanecarboxylic acid (4-methoxy phenyl)-amide), also known as WS-12.

Unless otherwise defined, all technical and scientific terms used herein have the same meaning as commonly understood by a person skilled in the art. Methods and materials similar or equivalent to those described herein can be used in the practice of the present invention. Throughout the description and claims the word "comprise" and its variations such as "comprising" are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

### EXAMPLES

### EXAMPLE 1: Formula for Composition-A

The formula for Composition-A is shown in Table 1:

**Table 1: Formula for composition A**

| Ingredients | Amount per formula and strength | Component / Function |
|---|---|---|
| Polyoxyl 35 castor oil (Cremophor EL) | 3.00 % | Non-ionic surfactant |
| Hypromellose (Methocel 4M) | 0.45 % | Thickening agent |
| NaH₂PO₄-2H₂O | 0.78 % | Buffer |
| NaCl | 0.55 % | Tonicity adjusting agent |
| NaOH 10 N, HCl 5 N | q.s.¹ pH 7.0 | pH adjusting agent |
| Purified water | q.s.¹ 100 % | Solvent |
| Total ophthalmic solution volume | 100 mL | |

| | | |
|---|---|---|
| ¹ q.s.: quantity sufficient | | |

The manufacturing process for Composition-A comprises the following steps:
Step 1: Methocel F4M solution was prepared using a 10 L glass reactor at 90 °C. Temperature and the stirring were kept until the Methocel F4M was completely dissolved.
Step 2: NaCl/NaH₂PO₄-2H₂O stock solution was prepared using a beaker and a magnetic stirrer. Stirring was kept at 1200 rpm until complete dissolution.
Step 3: Cremophor EL was heated at 60 °C and the solution was stirred until complete dissolution.
Step 4: Cremophor EL solution was added stepwise and under continuous stirring into NaCl/NaH₂PO₄-2H₂O stock solution. Then, pH was adjusted to 7.0 with NaOH 10 N. Finally, the solution was adjusted to 2000 g with water.
Step 5: Cremophor EL/NaCl/ NaH₂PO₄-2H₂O stock solution was added under continuous stirring into the Methocel F4M solution and 1 L of purified water previously cooled to 5 °C was added. The mixture was stirred during 20 minutes and he pH adjusted to 7.0 with HCl 5 N.
Step 6: Formulations were sterile filtered. The formulations were transferred into 20 L stainless steel pressure vessel, weighed and the final volume was adjusted to 800 g with water. The stainless steel pressure vessel was taken to the filling room and the filters were clamped to a discharge tube.
Step 7: Resulting ophthalmic solutions were packaged into single dose low-density polyethylene (LDPE) vials. With a syringe and a needle and in a vertical laminar flow booth the single dose LDPE vials were filled using the formulation stored on the sterile glass bottles. Every five single dose were packed into aluminum packets then thermally sealed.

### EXAMPLE 2: Formula for Composition-B

The formula for Composition-B is shown in Table 2:

**Table 2: Formula for composition B**

| Ingredients | Amount per formula and strength | Component / Function |
|---|---|---|
| Polyoxyl 35 castor oil (Cremophor EL) | 3.00 % | Non-ionic surfactant |
| WS-12 (on anhydrous base) | 0.0015 % | Active ingredient |
| Hypromellose (Methocel 4M) | 0.45 % | Thickening agent |
| NaH₂PO₄-2H₂O | 0.78 % | Buffer |
| NaCl | 0.55 % | Tonicity adjusting agent |
| NaOH 10 N, HCl 5 N | q.s.¹ pH 7.0 | pH adjusting agent |
| Purified water | q.s.¹ 100 % | Solvent |
| Total ophthalmic solution volume | 100 mL | |

| | | |
|---|---|---|
| ¹ q.s.: quantity sufficient | | |

The manufacturing process for Composition-B comprises the following steps:
Step 1 and Step 2 are the same than in EXAMPLE 1.
Step 3: Cremophor EL was heated at 60 °C. Then the corresponding amount of WS-12 was added stepwise and the solution was stirred until complete dissolution. WS-12/Cremophor EL solution was obtained.
Step 4-Step 7 are the same than in EXAMPLE 1 but with the WS-12/Cremophor EL solution.

### EXAMPLE 3: Formula for Composition-C

The formula for Composition-C is shown in Table 3:

**Table 3: Formula for composition C**

| Ingredients | Amount per formula and strength | Component / Function |
|---|---|---|
| Polyoxyl 35 castor oil (Cremophor EL) | 3.00 % | Non-ionic surfactant |
| Hyaluronic acid (m.m.w. ²) | 0.20 % | Active ingredient |
| Hypromellose (Methocel 4M) | 0.45 % | Thickening agent |
| NaH₂PO₄-2H₂O | 0.78 % | Buffer |
| NaCl | 0.55 % | Tonicity adjusting agent |
| NaOH 10 N, HCl 5 N | q.s.¹ pH 7.0 | pH adjusting agent |
| Purified water | q.s.¹ 100 % | Solvent |
| Total ophthalmic solution volume | 100 mL | |

| | | |
|---|---|---|
| ¹ q.s.: quantity sufficient ² m.m.w.: medium molecular weight (50kDa-500kDa) | | |

The manufacturing process for Composition-C comprises the following steps:
Step 1 is the same than in EXAMPLE 1.
Step 2: NaCl/NaH₂PO₄-2H₂O stock solution was prepared using a beaker and a magnetic stirrer. Then the corresponding amount of hyaluronic acid was added stepwise and stirring was kept at 800 rpm until complete dissolution.
Step 3: Cremophor EL was heated at 60 °C and the solution was stirred until complete dissolution.
Step 4: Cremophor EL solution was added stepwise and under continuous stirring into hyaluronic acid/NaCl/NaH₂PO₄-2H₂O stock solution. Then, pH was adjusted to 7.0 with NaOH 10 N. Finally, the solution was adjusted to 2000 g with water.
Step 5: Cremophor EL/hyaluronic acid/NaCl/NaH₂PO₄-2H₂O stock solution was added under continuous stirring into the Methocel F4M solution and 1 L of purified water previously cooled to 5 °C was added. The mixture was stirred during 20 minutes and he pH adjusted to 7.0 with HCI 5 N.
Step 6 and Step 7 are the same than in EXAMPLE 1.

### EXAMPLE 4: Clinical studies

A 4-week, randomized, double-masked clinical study was performed in mild to severe dry eye patients. Inclusion criteria for the study involved a baseline SANDE score ≥ 50, total staining (corneal and conjunctival) ≥ 1, Schirmer I Test score ≥ 3 mm to ≤ 9/5 min with anesthesia. SANDE assess the symptoms of dry eye disease through a subjective questionnaire; corneal and conjunctival staining assess damage of ocular surface and Schirmer I Test assess a measure tear production.

50 patients were randomized and distributed in two arms of treatment to receive Composition-A from EXAMPLE 1 in two different dosing regimens (BID: twice a day and TID: three times a day) for 28 days. In particular, 23 patients were randomized to the Composition-A BID treatment, and 27 patients were randomized to the Composition-A TID treatment. Each treatment administration started after 1-5 days wherein artificial tears or other dry eye disease therapy were suppressed. Symptoms (SANDE) and signs of dry eye disease (corneal and conjunctival staining) were evaluated at days 0 (baseline), 7, 14 and 28.

### EXAMPLE 4.1: Symptoms of dry eye disease evaluation

The SANDE questionnaire is a validated global approach to assess the symptoms of dry eye disease based in determining the frequency and severity of the symptoms according to patient subjective response. A global score is obtained from multiplying the frequency and severity values and obtaining the square root (Schaumberg DA. et al., 2007). This tool has been proved to be a reliable and valid measure in patients with dry eye symptoms.

FIG. 1 (1A, 1B and 1C) shows a significant decrease regarding SANDE scores from baseline as early as 7 days of treatment with Composition-A. Similar decreases are observed in both treatments (BID and TID) meaning that Composition-A, independently of the posology, is associated with a significant decrease in SANDE scores which involves a significant improvement in symptoms of dry eye. Differences from baseline to day 28 of treatment achieve a solid statistical significance of p value p<0.0001. Detailed values are shown in Table 4.

More in detail, FIG. 1A shows the treatment effect of Composition-A in SANDE total scores: after 7 days of treatment the reduction in SANDE scores from baseline was 35 % and 30 % for BID and TID, respectively. FIG. 1B shows the treatment effect of Composition-A in SANDE frequency scores: after 7 days of treatment the reduction in SANDE scores from baseline was 36 % and 24 % for BID and TID, respectively. FIG. 1C shows the treatment effect of Composition-A in SANDE severity scores. After 7 days of treatment the reduction in SANDE scores from baseline was 31 % and 30 % for BID and TID, respectively. The reduction on all three SANDE scores was maintained at 28 days of treatment.

**Table 4: Effect of Composition-A in SANDE score**

| SANDE | Regimens | Baseline | Day 7 | Day 14 | Day 28 |
|---|---|---|---|---|---|
| Total | BID | 66.43 ± 12.95 | 43.18 ± 22.95 | 43.40 ± 24.84 | 41.37 ± 22.49 |
| | TID | 74.87 ± 16.25 | 52.08 ± 21.96 | 46.82 ± 25.00 | 43.80 ± 25.68 |
| Frequency | BID | 69.77 ± 15.61 | 44.73 ± 25.05 | 44.96 ± 25.63 | 41.58 ± 23.18 |
| | TID | 74.62 ± 16.35 | 56.87 ± 20.47 | 51.77 ± 23.19 | 46.87 ± 24.30 |
| Severity | BID | 64.32 ± 15.24 | 44.53 ± 23.04 | 43.09 ± 25.35 | 41.94 ± 22.12 |
| | TID | 75.41 ± 17.23 | 52.98 ± 21.20 | 45.48 ± 29.00 | 43.07 ± 27.88 |

### EXAMPLE 4.2: Signs of dry eye disease evaluation

Corneal and conjunctival staining assess damage of ocular surface by staining the cornea with fluorescein dye (I-DEW FLO, Entod Research Cell UK Ltd) and conjunctiva with lissamine green dye (I-DEW GREEN, Entod Research Cell UK Ltd). Per eye, 2 µL of 2 % sterile fluorescein was instilled into the conjunctival sac and the upper eyelid was lifted slightly to grade the whole cornea surface. Since fluorescein diffuses rapidly into tissues, punctuate staining blurs after a short period. It is therefore essential to assess staining rapidly, in sequence, in the right and then the left eye, so that the staining patterns observed are equally crisp.

After recovery, 25 µL of 1 % lissamine green was instilled onto the upper bulbar conjunctiva with the upper lid retracted and the patient looking down. Both eyes may be stained prior to grading, since there is no risk of the staining pattern in the first eye being obscured by the time the second eye is graded. The subject must look nasally to grade the temporal zone and temporally to grade the nasal zone.

Corneal staining with fluorescein and conjunctival staining with lissamine green was graded according to Oxford scale (Bron et al., 2003 and Craig JP et al, 2017).

FIG. 2A shows the treatment effect of Composition-A on corneal fluorescein staining. There is a consistent slope in reduction of staining for both treatments (BID and TID) from day 1 to day 28, suggesting a therapeutic effect of Composition-A in improving corneal surface damage. In this case, TID treatment exhibits a statistically significant reduction of corneal fluorescein staining at day 28 from baseline with a p value of 0.0004.

FIG. 2B shows the treatment effect of Composition-A on conjunctival lissamine staining. There is a consistent slope in reduction of staining for both treatments (BID and TID) from day 1 to day 28, suggesting a therapeutic effect of Composition-A in improving conjunctival surface damage. In this case, TID treatment exhibits a statistically significant reduction of corneal fluorescein staining at day 28 from baseline with a p value of 0.005. Detailed values are shown in Table 5.

**Table 5: Effect of Composition-A in staining**

| Staining | Regimens | Baseline | Day 7 | Day 14 | Day 28 |
|---|---|---|---|---|---|
| Corneal | BID | 1.39 ± 0.72 | 1.10 ± 0.68 | 0.97 ± 0.75 | 1.00 ± 0.73 |
| | TID | 1.53 ± 0.97 | 1.38 ± 0.97 | 1.20 ± 0.98 | 0.83 ± 1.05 |
| Conjunctival | BID | 1.15 ± 0.93 | 0.86 ± 0.77 | 0.90 ± 1.05 | 0.65 ± 0.87 |
| | TID | 1.09 ± 0.94 | 1.00 ± 1.05 | 0.92 ± 1.05 | 0.48 ± 0.72 |

### REFERENCES

### Non-patent literature:

Lee JE et al., "Effect of Cyclosporine A 0.05% on Human Corneal Epithelial Cells" J Korean Ophthalmol Soc 2007, vol. 48(10) pp. 1399-1409
Garweg JG et al., " Effects of daunorubicin, mitomycin C, azathioprine and cyclosporin A on human retinal pigmented epithelial, corneal endothelial and conjunctival cell lines" Graefe's Arch Clin Exp Ophthalmol 2006 vol. 244 pp. 382-389
Craig JP et al., "TFOS DEWS II Definition and Classification Report" The Ocular Surface 2017 vol. 15 pp. 276-283
Schaumberg DA. et al., "Development and validation of a short global dry eye symptom index." The Ocular Surface 2007 vol. 5 pp. 4533-4544
Bron et al., "Grading Of Corneal and Conjunctival Staining in the Context of Other Dry Eye Tests" Cornea 2003 vol. 22 pp. 640-650

## Claims

1. An ophthalmic composition comprising a non-ionic surfactant as active ingredient, wherein the non-ionic surfactant is present in an effective amount from 0.01 to 8 weight/volume % based on the total composition, for use in treating or preventing dry eye and/or for relieving one or more symptoms and/or signs of dry eye.

2. The ophthalmic composition according to claim 1, wherein the symptoms and/or signs are selected from the group consisting of: foreign body sensation, burning, stinging, itching, pain, dryness, redness of the eye, reduced tear volume, tear poor quality, reduced tear break-up time, presence of small dried areas in the cornea and conjunctiva detected by staining.

3. The ophthalmic composition according to any of claims 1-2, wherein the non-ionic surfactant is selected from the group consisting of an oleoyl macrogolglyceride, a linoleoyl macrogolglyceride, a caprylocaproyl polyoxylglyceride, polyoxyl 35 castor oil, polyoxyl 35 hydrogenated castor oil, polyoxyl 40 castor oil, polyoxyl 40 hydrogenated castor oil, and a condensation product of ethylene oxide with 12-hydroxystearic acid.

4. The ophthalmic composition according to claim 3, wherein the non-ionic surfactant is polyoxyl 35 castor oil.

5. The ophthalmic composition according to any of claims 1-4, wherein the non-ionic surfactant is present in an amount from 0.5 to 5 w/v %.

6. The ophthalmic composition according to claim 5, wherein the non-ionic surfactant is present at 3 w/v %.

7. The ophthalmic composition according to any of claims 1-6, wherein dry eye or the symptoms and/or signs thereof, are caused or associated to a disease or condition selected from the group consisting of keratoconjunctivitis sicca, keratoconjunctival epithelial disorder, reduced lacrimal fluid secretion, Stevens-Johnson syndrome, Sjogren's syndrome, tear deficiency, ocular hyperemia and, tear film instability.

8. The ophthalmic composition according to any of claims 1-6, , wherein dry eye or the symptoms and/or signs thereof, are caused or associated to a factor selected from the group consisting of contact lenses use, digital device use, refractive or other ocular surgery, chronic administration of neurotropic drugs, allergic, amoebal and viral keratoconjunctivitis, aging, hormonal changes, and environmental factors.

9. The ophthalmic composition according to any of claims 1-8, wherein the ophthalmic composition is formulated as eye drops.

10. The ophthalmic composition according to any of claims 1-9, further comprising a buffer.

11. The ophthalmic composition according to any of claims 1-10, further comprising a second active ingredient.

12. The ophthalmic composition according to claim 11, wherein the second active ingredient is selected from the group consisting of a Transient Receptor Potential cation channel subfamily M member 8 (TRPM8) agonist, and hyaluronic acid or a salt thereof.

13. The ophthalmic composition according to claim 12, wherein the second active ingredient is a TRPM8 agonist and is present in an amount from 0.0001 to 0.1 w/v %.

14. The ophthalmic composition according to any of claims 12-13, wherein the second active ingredient is the TRPM8 agonist 2-isopropyl-5-methyl-cyclohexanecarboxylic acid (4-methoxy phenyl)-amide (WS-12).

15. An ophthalmic composition consisting of a non-ionic surfactant as sole active ingredient, a buffer, a tonicity adjusting agent and a pH adjusting agent, wherein the non-ionic surfactant is present in an effective amount from 0.01 to 8 w/v % based on the total composition.
